# EUROPEAN PATENT APPLICATION

(11) **EP 1 400 538 A1**
(43) Date of publication of application: **24.03.2004**
(21) Application number: 03292153.8
(22) Date of filing: 02.09.2003
(51) Int. Cl.: C08F 2/10, C08F 2/48, A61K 7/00

(54) **Method of producing synthetic polymer gel**

(30) Priority: 11.09.2002 JP 2002265123
(71) Applicant: L'OREAL, 75008 Paris (FR)
(72) Inventor: Ikkai, Fumiyoshi, Ibaraki (JP)
(74) Representative: Dodin, Catherine

(57) **Abstract**

The present invention concerns a method of producing a synthetic polymer gel comprising the steps of preparing an aqueous solution containing a persulfate and a water-soluble vinyl monomer, then irradiating said aqueous solution with UV light.

## Description

The present invention relates to a method of producing a synthetic polymer gel. More specifically, the present invention relates to a method for very easily obtaining a hydrogel by UV irradiation of an aqueous solution containing a persulfate and a water - soluble vinyl monomer having a specific structure.

Synthetic polymer gels, particularly hydrogels, can serve a variety of functions based on properties such as water retention property and flexible and elastic property as well as their ability to provide controlled release of encapsulated agents, and are therefore used for diverse applications in fields such as medicine and hygiene, cosmetics, food processing, machinery, construction and agriculture.

Hydrogels composed of synthetic polymers are believed to be formed by insoluble three-dimensional network structures made up of constituent synthetic polymer chains that are swollen with water. In these three-dimensional network structures, polymer chains bind together by covalent bonds, hydrogen bonds, coordinate bonds or Coulomb bonds, and groups of polymer chains are coagulated or intertwined together. In particular, hydrogels composed of synthetic polymers crosslinked by covalent bonds can be employed for a wide range of uses due to their good stability. The production of such synthetic polymer gels due to covalent bonds has principally been prepared by (1 ) methods in which crosslinking progresses simultaneous to polymerisation of monomers, and (2) methods of generating crosslinked bonds between polymerised macromolecular chains.

In general, polymerisation reactions are made up of a number of elementary reactions including an initiation reaction, a propagation reaction, a chain transfer reaction and a termination reaction, and in the initiation reaction for radical polymerisation, a radical-generating compound known as an initiator is generally used.
When energy in the form of heat or light is applied to a monomer solution containing an initiator, the initiator will cleave to create radicals, and these radicals will attack (vinyl) monomer double bonds to generate monomer radicals, which will in turn attack subsequent monomers. This chain reaction will continue to propagate and build up polymer chains until a chain transfer reaction to another molecule or a termination reaction such as disproportionation finally causes the polymerisation to cease.

If at least two types of monomer exist in the polymerisation solution, the different types of monomer in the propagation reaction will bind together to form copolymers. In this case, differences in the reactivity (copolymerizability) of the existing monomers can cause differences in the proportion of monomers forming the final polymer chains or prevent the polymerisation from proceeding far enough.
In the above-described method (1 ), crosslinking agents such as divinyl compounds or the like are usually added to the monomer solution to induce a crosslinking reaction simultaneous to the polymerisation of the monomers. In this case also, the reactivity of the monomers and crosslinking agents being used must be adjusted in order to obtain the desired crosslinked polymer.

Furthermore, in emulsion polymerisation wherein a monomer solution is dispersed in the medium, there are cases in which raising the temperature of the medium will disrupt the balance at the boundary and make it impossible to maintain a good state of dispersion. Consequently, in emulsion polymerisation, a low-temperature initiator (redox initiator) containing a peroxide which decomposes in the presence of a reducing agent is often used.
This redox initiator is a combination of an oxidizing agent such as a persulfate or hydrogen peroxide, and an inorganic reducing agent such as a water-soluble ferrous salt or NaHSO₃ or an organic reducing agent such as alcohol or polyamine. By using this initiator, radicals are formed by an electron transfer from the reducing agent to the oxidizing agent, and these radicals initiate the polymerisation reaction of the monomers. However, there are cases in which an emulsive state cannot be maintained due to action of the reducing agent contained in the redox initiator on the surfactant used to form the emulsion.

On the other hand, in the above-described method (2), reactive functional groups must be preattached to the polymer chains in order to enable to occur a crosslinking reaction between the polymer chains, but there is the risk of unreacted functional groups that remain after crosslinking having a detrimental effect on the properties of the gel.

Therefore, there has been a demand for a method of producing a gel which, particularly in the case of emulsion polymerisation, does not have a detrimental effect on the state of emulsion, and is also able to conveniently form crosslinked synthetic polymers gel.
There has also been a demand for a method of producing a hydrogel without using crosslinking agents or reducing agents of low-temperature redox initiators as used in conventional gelation, for example.

The above-described problems are all resolved by the present invention as explained below.
That is, the present invention is a method of producing a synthetic polymer gel comprising steps of preparing an aqueous solution containing a persulfate and at least one type of water-soluble vinyl monomer having an acryloyl (CH2=CHCO-) structure, and irradiating said aqueous solution with UV light to form a gel.

Another object of the present invention is a method of producing coated particles comprising steps of (i) formulating an aqueous solution comprising at least a persulfate, at least one type of water-soluble vinyl monomer having an acryloyl structure, and at least a pigment, filler and/or nacre, (ii) dispersing said aqueous solution in a water-immiscible medium to form a suspension or water-in-oil type emulsion, (iii) gelling said suspension or water-in-oil emulsion by irradiation with UV light to form a synthetic polymer gel particles dispersion and (iv) separating the synthetic polymer gel particles from the medium, to obtain coated particles comprising a core consisting of pigment, filler and/or nacre particle and synthetic polymer gel as a shell.

Another object is the synthetic polymer gel, the dispersion of synthetic polymer gel particles, the synthetic polymer gel particle and the coated particle susceptible to be produced by this method.

Another object is a cosmetic composition comprising, in a cosmetically acceptable medium, said synthetic polymer gel, said dispersion of synthetic polymer gel particles, said synthetic polymer gel particle and/or said coated particle.

By using the method of the present invention, crosslinking agents and redox initiators, as well as gamma(y)-ray treatments, which are generally used for the production of conventional synthetic polymer gels, are unnecessary, so that a synthetic polymer gel can be produced very conveniently and stably.
According to the present invention, it is possible to gelate an aqueous solution containing no additives such as crosslinking agents or redox initiators, resulting in a pure hydrogel consisting essentially of crosslinked polymer consisting of the vinyl monomers. However, this does not mean preclude the addition of any other additives such as those described later.

In the method of producing a synthetic polymer gel (hydrogel) according to the present invention, a water-soluble compound believed to function as an initiator, in particular, an aqueous solution of a persulfate and a water-soluble vinyl monomer having the above-described specific structure is first prepared.

The vinyl monomers which are used are not particularly restricted as long as they have an acryloyl structure and are water-soluble, but it is preferable to use those having a structure expressed by the following formula (I):

CH₂=CHCO-R (I)

wherein R denotes an arbitrary substituant group selected from the group consisting of - OH, -R1, -OR1, -NH2, -NH-R1 or-N-R1R2,
in which R1 and R2 which may be identical or different, represent a straight or branched or cyclic alkyl group or alkoxyl group having 1-10, preferably 1-8, more preferably 1-6, still more preferably 1-4 carbon atoms which may optionally contain a hetero atom such as O, S or N in the chain and may optionally substituted with one or more groups such as halogen atom, hydroxyl group, carboxyl group, formyl group and/or amino group.
Preferably, R could be OH, O(CH₂)₂N(CH₃)₂, O(CH₂)₂OH, NH₂, NHC₃H₇, N(CH₃)₂.

The term "water-soluble" as used herein is understood as meaning soluble in water without heating (at 25°C), at a concentration of 1% by weight.
Among the water-soluble monomers usable in the present invention, mention may be made of acrylic acid; acrylamide; N-alkylacrylamide, such as N-isopropylacrylamide, N-hexylacrylamide, N-ethylacrylamide and N-methylacrylamide; N,N-dialkylacrylamide, such as N,N-diisopropylacrylamide, N,N-dihexylacrylamide, N,N-diethylacrylamide, N,N-dimethylacrylamide, N-methyl-N-ethylacrylamide, N-ethyl-N-hexylacrylamide and 4-acryloylmorpholine; acrylic esters including alkyl acrylate, preferably C1-C12alkyl acrylate, such as isopropylacrylate, hexylacrylate, ethylacrylate, 2-(dimethylamino)ethylacrylate and 2-hydroxyethylacrylate, and mixtures thereof.

The concentration of the water-soluble vinyl monomer is generally 300-1200 mM, preferably 500-1000 mM, more preferably 700-800 mM in the aqueous solution to be gelled. However, depending on the conditions such as the polymerisation system (solution polymerisation, suspension polymerisation or emulsion polymerisation), or the desired degree of polymerisation or crosslinking (or gelation), or the concentration of the persulfates being used, a concentration outside the above-given range may also be used.

The aqueous solution prepared herein contains at least one type of persulfate in addition to the aforementioned water-soluble vinyl monomer.
The persulfates that are used are preferably selected from among ammonium persulfate, potassium persulfate and sodium persulfate, and mixtures thereof, but are not restricted thereto. Persulfates are substances which are usually used in combination with reducing agents as the above-mentioned redox initiators.
However, in the present invention, the persulfates are used alone without using any reducing agents.
These persulfates are believed to act as polymerisation initiators by cleaving when later irradiated with UV light. As far as the present inventors know, there are no reports relating to the optical properties of persulfates.
These persulfates are generally used in the aqueous solution at concentrations of 0.01-1.0 wt%, preferably 0.1-0.5 wt%, and more preferably 0.2-0.3 wt%.
However, as with the concentration of the water-soluble vinyl monomer, the concentration may be outside this range depending on the conditions or uses thereof.

What deserves special mention is that, in the present invention, the persulfates which are conventionally used as the oxidizing agents in the redox initiator are used for polymerisation by light (UV radiation). The photopolymerization initiators which are conventionally used include compounds which are photocleaved to generate radicals such as benzoin ethers, acyl phosphine oxides and acyl phosphonates, and compounds which generate radicals by hydrogen drawing or by electron transfer such as benzophenones, most of these being water-insoluble compounds having a benzene ring structure and often having toxicity or colour. On the other hand, the persulfates used in the present invention are very highly water-soluble, and are safe to use and colourless. Therefore, when producing the synthetic polymer hydrogel, the concentration of the persulfates can be adjusted over a wide range, thereby making it possible to easily adjust the speed of gelation, and the molecular weight and degree of crosslinking of the polymer.

The above-described persulfates and water-soluble vinyl monomers can generally be dissolved in water at room temperature.
While this aqueous solution may contain arbitrary ingredients other than the persulfates and water-soluble vinyl monomers depending on the uses thereof, such arbitrary components must of course be used with care such as not to inhibit the gelation due to the present invention.
Among such arbitrary components, it is possible to mention pigments, nacres and/or fillers such as those listed later in the specification.

The prepared aqueous solution could be placed in a UV-permeable container of preferably silica or the like, and the aqueous solution is irradiated with UV light.
While UV light is generally divided by wavelength into the UV-A region (wavelength about 320-400 nm), the UV-B region (wavelength about 290-320 nm) and the UV-C region (wavelength about 200-290 nm), the present invention most preferably uses UV light in the UV-C region, especially UV light of short wavelengths of about 200-250 nm. The UV light irradiation time may differ according to the concentrations of persulfates and/or water-soluble vinyl monomers contained in the aqueous solution and the desired gelation rate, but is generally 1-60 minutes, preferably 3-45 minutes, more preferably 5-30 minutes.

By performing the above operations, polymerisation and crosslinking reactions will proceed in the aqueous solution in the container, which will gradually lose fluidity until the entire aqueous solution gels.
Aside from the crosslinked synthetic polymers and the aforementioned arbitrary ingredients, the synthetic polymer gel produced in this way does not contain any extraneous ingredients such as reducing agents contained in conventional redox initiators.

In a specific embodiment of the method of the present invention, an aqueous solution containing persulfates and water-soluble vinyl monomers as well as the aforementioned arbitrary ingredients such as pigments, nacres and/or fillers, shall be prepared as described above. Next, the solution is dispersed in a water-immiscible medium, for example, an organic solvent such as ethyl acetate, dichloromethane, petroleum ether or benzene, and/or an oil such as liquid paraffin, vegetable oil, animal oil, synthetic oil or mineral oil.
Mention may be made in particular of:
- hydrocarbon-based oils of animal origin;
- hydrocarbon-based plant oils;
- linear or branched hydrocarbons of mineral or synthetic origin;
- synthetic esters and ethers of fatty acids;
- fatty alcohol containing from 12 to 26 carbon atoms; and
- mixtures thereof.

This operation includes both an operation of adding the aforementioned medium to the aqueous solution and stirring with a homogeniser or the like to form a suspension, and an operation of using an appropriate surfactant (emulsifier) to emulsify the aqueous particles in the medium to form a water-in-oil type emulsion. The surfactant could be a ester of sorbitane or of glycerol; and alkylester or alkylether of sorbitane or glycerol.
Next, the suspension or emulsion could be placed in a UV-transparent container as described above, and similarly irradiated with UV light.

As a result, a dispersion in the medium of synthetic polymer gel particles, preferably particles of a uniform size, can be obtained quite easily.
Since the method of the present invention does not use a reducing agent such as in a conventional redox initiator, it is able to produce dispersed synthetic polymer gel particles by simply irradiating with UV light, without disrupting the balance of the emulsion system.

The synthetic polymer gel particles created in this way can be separated from the medium as needed in accordance with methods known in the relevant field. For example, an operation of repeatedly adding a volatile organic solvent such as hexane to the medium containing the generated synthetic polymer gel particles and removing the supernatant fluid, and finally removing the solvent by natural drying or freeze-drying can be used to obtain synthetic polymer gel particles. Depending on the situation, a method of putting the medium in which the particles are dispersed under centrifugation to precipitate the particles and removing the supernatant fluid may also be used. Additionally, particularly in the case of synthetic polymer gel particles of large size, the medium in which the particles are dispersed can be spread out over a mesh with pores smaller than the particle size to remove the medium, then further cleansed with hexane or the like.

When performing the method of the present invention on an aqueous solution containing persulfates and water-soluble vinyl monomers, as well as the aforementioned arbitrary ingredients, and further containing pigments, fillers and/or nacre particles dispersed in the medium; gelling the aqueous solution or dispersion by UV-irradiation, for example by placing it in a UV-transparent container as described above, and similarly irradiating with UV light, then separating the gelled particles for example using the above mentioned methods of separation, it is possible to obtain pigment, nacre or filler particles having their surfaces protected by a synthetic polymer gel. Said particles could be considered as 'coated particles'.

These coated particles comprising pigment, filler and/or nacre as a core and synthetic polymer gel as a shell are especially useful in fields such as cosmetics.

The term "pigments" should be understood as meaning white or colored, mineral or organic particles intended to colour and/or opacify the composition. The term "fillers" should be understood as meaning colourless or white, mineral or synthetic, lamellar or non-lamellar particles intended to give body or rigidity to the composition, and/or softness, a matt effect and uniformity to the make-up result. The term "nacres" should be understood as meaning iridescent particles which reflect light.
The pigments can be white or colored, mineral and/or organic, of micrometric or nanometric size. Mineral pigments which may be mentioned include titanium dioxide, zirconium dioxide or cerium dioxide, and also zinc oxide, iron oxide or chromium oxide and ferric blue. Organic pigments which may be mentioned include carbon black and barium, strontium, calcium and aluminium lakes.
Among the nacres which may be envisaged, mention may be made of mica coated with titanium oxide, with iron oxide, with natural pigment or with bismuth oxychloride, and also colored titanium mica.
The fillers can be mineral or synthetic, and lamellar or non-lamellar. Mention may be made of talc, mica, silica, kaolin, Nylon powder, polyethylene powder, Teflon, starch, titanium mica, natural mother-of-pearl, boron nitride, microspheres such as Expancel (Nobel Industrie), Polytrap (Dow Corning) and silicone resin microbeads (Tospearls from Toshiba, for example).

The synthetic polymer gel, the synthetic polymer gel particles, the coated particles, the dispersion comprising said synthetic polymer gel particles and/or the dispersion comprising said coated particles, could be advantageously used in cosmetic compositions.
it is preferably present in the composition in an amount which may readily be determined by a person skilled in the art depending on the desired effect, and which may be between 0.1 % and 40% by weight, for example between 1% and 15% by weight, relative to the total weight of the composition, and better still between 24% and 12% by weight, or even between 4% and 10% by weight of the composition.

The cosmetic composition according to the invention could comprise a cosmetically or dermatologically acceptable medium that could comprise at least one cosmetically or dermatologically acceptable oil, i.e. a fatty substance which is liquid at room temperature (25°C). These oils can be hydrocarbon-based oils and/or silicone oils and/or fluoro oils. They can be of animal, plant, mineral or synthetic origin. The oils used can be volatile and/or non-volatile.
Mention may be made in particular of:
- hydrocarbon-based oils of animal origin such as perhydrosqualene;
- hydrocarbon-based plant oils such as liquid triglycerides of fatty acids of 4 to 10 carbon atoms, for instance heptanoic or octanoic acid triglycerides; sunflower oil, corn oil, soybean oil, marrow oil, grapeseed oil, groundnut oil, sweet almond oil, beauty-leaf oil, palm oil, sesame oil, hazelnut oil, apricot oil, macadamia oil, castor oil, avocado oil; caprylic/capric acid triglycerides, jojoba oil, karite butter;
- linear or branched hydrocarbons of mineral or synthetic origin, such as liquid paraffins and derivatives thereof, petroleum jelly, polydecenes, and hydrogenated polyisobutene such as parleam;
- synthetic esters and ethers, in particular of fatty acids, for instance the oils of formula R3COOR4 in which R3 represents a higher fatty acid residue containing from 7 to 29 carbon atoms and R4 represents a hydrocarbon-based chain containing from 3 to 30 carbon atoms, such as, for example, purcellin oil (cetostearyl ocatanoate), isopropyl myristate, 2-ethylhexyl palmitate, 2-octyldodecyl stearate, 2-otyldodecyl erucate, isostearyl isostearate; hydroxylated esters such as isostearyl lactate, octyl hydroxystearate, octyldodecyl hydroxystearate, diisostearyl malate, triisocetyl citrate, and fatty alkyl heptanoates, octanoates and decanoates; polyol esters, for instance propylene glycol dioctanoate, neopentyl glycol diheptanoate or diethylene glycol diisononanoate; and pentaerythritol esters; tridecyl trimellitate;
- fatty alcohols containing from 12 to 26 carbon atoms, for instance octyldodecanol, 2-butyloctanol, 2-hexyldecanol, 2-undecylpentadecanol or oleyl alcohol;
- partially hydrocarbon-based and/or silicone-containing fluoro oils;
- silicon oils, for instance volatile or non-volatile, linear or cyclic polymethylsiloxanes (PDMS); alkyldimethicones; phenylsilicone oils;
- mixtures thereof.

The composition could also comprises at least a wax that could be natural waxes of animal, plant or mineral origin, such as beeswax, montan wax, carnauba wax, Candellila wax, China wax, flax wax, pine wax, cotton wax, Ouricury wax, lignite wax, rice bran wax, sugar cane wax, Japan wax, or cork fibre wax; paraffin waxes, microcrystalline waxes, lanolin wax, ozokerites, hydrogenated oils with a melting point of greater than about 40°C, for instance hydrogenated jojoba oil, polyethylene waxes derived from the polymerisation of ethylene, waxes obtained by Fischer-Tropsch synthesis, fatty acid esters and glycerides with a melting point of greater than about 40°C; silicone waxes.

The composition according to the invention can moreover comprise one or more constituents usually used in the type of application envisaged such as:
- organic solvents chosen in particular from ketones, alcohols; glycols; propylene glycol ethers; short-chain esters (containing from 3 to 8 carbon atoms in total);ethers; alkanes.
- water, alone or in combination with hydrophilic active agents such as moisturizers, and/or hydrophilic gelling agents.
- surfactants,
- polymers, film-forming or not;
- dyestuff which can be chosen from the lipophilic dyes, hydrophilic dyes, pigments, nacres, usually used in cosmetic compositions, and mixtures thereof.
- additive usually used in cosmetics, such as fillers, antioxidants, fragrances, essential oils, preserving agents, cosmetic active agents, vitamins, essential fatty acids, sphingolipids, self-tanning compounds such as DHA, sunscreens.

The compositions according to the invention are intended to be applied to the skin of the face and of the body, to mucous membranes and/or to keratin fibres such as the nails, the eyelashes or the hair.
They can be in any conceivable cosmetic form, such as a solid or soft oily gel, optionally comprising water; a solid or gelled oil-in-water, water-in-oil or multiple emulsion; a dispersion of oil in water; a multi-phase system and in particular a two-phase system. They can have the appearance of a cream, a salve, a soft paste, an ointment, a cast or moulded solid and in particular a stick. They can be in particular in the form of a stick or a dish; and in particular in the form of a transparent anhydrous rigid gel, and more especially in the form of a translucent or transparent anhydrous stick.

These compositions find an application in particular as body hygiene compositions, for example in the form of deodorant sticks; as a hair composition, for example as a styling stick or a make-up stick for the hair; as a make-up composition for the skin of the face or the body or for mucous membranes, for example as a lipstick, a foundation cast as a stick or a dish, a face powder, an eyeshadow, a fixing base to be applied over a conventional lipstick, a concealer stick, a lip gloss, an eyeliner, a mascara or temporary tattoo products; as a care composition for the skin or mucous membranes, for example as a lipcare balm or base, an ointment for the body or a dally care cream; as an antisun composition or a self-tanning composition; as skin-care compositions such as creams or facial washing gel.

Herebelow, the present invention shall be described in further detail by giving specific examples.

### Examples 1-7 and Comparative Examples 1-7

Aqueous solutions comprising 700-800 mM of the respective water-soluble vinyl monomers indicated in Table 1 and 0.2-0.3 wt% of ammonium persulfate were prepared.
The water-soluble vinyl monomers which were used had structures expressed by the following formula (II): CH₂=CRaRb (II).

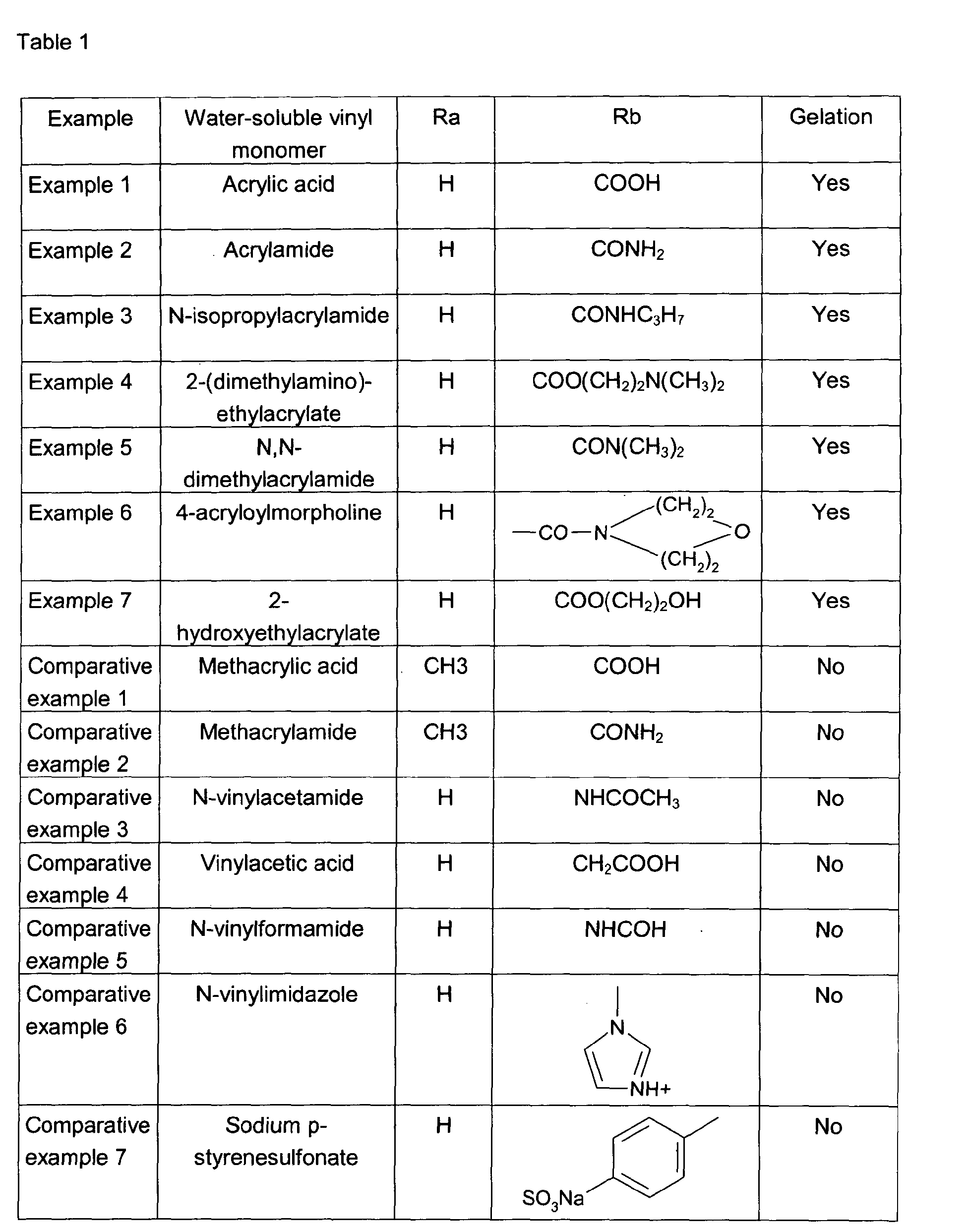

1 ml of each aqueous solution was placed in a silica cell of thickness 4 mm and width 1 mm, and irradiated for 5-30 minutes with UV light from a 500 W mercury lamp from a distance of about 20 cm.

The aqueous solutions of Examples 1-7 did not run even when the silica cells were tilted after irradiation with UV rays, whereas the aqueous solutions of Comparative Examples 1-7 maintained fluidity even after UV irradiation.
These results are indicated in Table 1 by "yes" for those which lost fluidity (= gelation) and "no" for those which maintained fluidity (no gelation).
When irradiated under the same conditions via a filter for blocking UV light of wavelength 250 nm or less, the aqueous solutions of Examples 1-7 maintained their fluidity (gelation did not occur).

In the Examples, the gelation (loss of fluidity) was measured by tilting the silica cell by 180 degrees (inverting the cell) and observing the material in the cell. If the materials did not move after several (2) minutes, we determined that the materials were gelled (lost their fluidity); alternatively, if the materials moved, we determined that the materials had not gelled (maintained their fluidity).
Accordingly, a strict benchmark was employed in these examples in which deformable materials such as soft gels or viscous fluid were not considered to be gels in the examples. However, the process of the present invention can also produce such deformable gels, and manufacture of such deformable gels is included within scope of the present invention.

## Claims

1. A method of producing a synthetic polymer gel comprising steps of formulating an aqueous solution comprising at least a persulfate and at least one type of water-soluble vinyl monomer having an acryloyl structure, then gelling said aqueous solution by irradiation with UV light.

2. A method in accordance with claim 1, wherein the water-soluble vinyl monomer has a structure expressed by the following formula (I):
CH2=CHCO-R (I)
wherein R denotes a substituant group selected from the group consisting of -OH, -R1, - OR1, -NH2, -NH-R1 or-N-R1R2,
in which R1 and R2 which may be identical or different, represent a straight or branched or cyclic alkyl group or alkoxyl group having 1-10, preferably 1-8, more preferably 1-6, still more preferably 1-4 carbon atoms which may optionally contain a hetero atom such as O, S or N in the chain and may optionally substituted with one or more groups such as halogen atom, hydroxyl group, carboxyl group, formyl group and/or amino group.

3. A method in accordance with any one of claims 1-2, wherein the water-soluble vinyl monomer is selected from acrylic acid; acrylamide; N-alkylacrylamide, such as N-isopropylacrylamide, N-hexylacrylamide, N-ethylacrylamide and N-methylacrylamide; N,N-dialkylacrylamide, such as N,N-diisopropylacrylamide, N,N-dihexylacrylamide, N,N-diethylacrylamide, N,N-dimethylacrylamide, N-methyl-N-ethylacrylamide, N-ethyl-N-hexylacrylamide and 4-acryloylmorpholine; acrylic esters including alkyl acrylate, preferably C1-C12alkyl acrylate, such as isopropylacrylate, hexylacrylate, ethylacrylate, 2-(dimethylamino)ethylacrylate and 2-hydroxyethylacrylate, and mixtures thereof.

4. A method in accordance with any one of claims 1-3, wherein the concentration of the water-soluble vinyl monomer in the aqueous solution is 300-1200 mM, preferably 500-1000 mM, more preferably 700-800 mM.

5. A method in accordance with any one of claims 1-4, wherein the persulfate is selected from the group consisting of ammonium persulfate, potassium persulfate and sodium persulfate and mixtures thereof.

6. A method in accordance with any one of claims 1-5, wherein the concentration of the persulfate in the aqueous solution is 0.01-1.0 wt%, preferably 0.1-0.5 wt%, and more preferably 0.2-0.3 wt%.

7. A method in accordance with any one of claims 1-6, wherein the aqueous solution further comprise pigments, fillers and/or nacres.

8. A method in accordance with any one of claims 1-7, wherein the wavelength of the UV light is in the UV-C region (wavelength about 200-290 nm), especially UV light of short wavelengths of about 200-250 nm.

9. A method in accordance with any one of claims 1-8, wherein the irradiation time is 1-60 minutes, preferably 3-45 minutes, more preferably 5-30 minutes.

10. A method in accordance with any one of claims 1-9, further comprising a step, after the step of formulating said aqueous solution and before the step of irradiating with UV light, of dispersing said aqueous solution in a water-immiscible medium to form a suspension or water-in-oil type emulsion, and said suspension or water-in-oil emulsion is irradiated with UV light to form a synthetic polymer gel particles dispersion.

11. A method in accordance with claim 10, wherein the water-immiscible medium is selected from an organic solvent such as ethyl acetate, dichloromethane, petroleum ether or benzene; an oil such as liquid paraffin, vegetable oil, animal oil, synthetic oil or mineral oil; mixtures thereof.

12. A method in accordance with any one of claims 10-11, further comprising a step of separating the synthetic polymer gel particles from the medium, to obtain synthetic polymer gel particles.

13. A method of producing coated particles comprising steps of (i) formulating an aqueous solution comprising at least a persulfate, at least one type of water-soluble vinyl monomer having an acryloyl structure, and at least a pigment, filler and/or nacre, (ii) dispersing said aqueous solution in a water-immiscible medium to form a suspension or water-in-oil type emulsion, (iii) gelling said suspension or water-in-oil emulsion by irradiation with UV light to form a synthetic polymer gel particles dispersion and (iv) separating the synthetic polymer gel particles from the medium, to obtain coated particles comprising a core consisting of pigment, filler and/or nacre particle and synthetic polymer gel as a shell.

14. A synthetic polymer gel susceptible to be produced by the method of any one of claims 1-9.

15. A dispersion of synthetic polymer gel particles susceptible to be produced by the method of claim 10.

16. A synthetic polymer gel particle susceptible to be produced by the method of claim 12.

17. A coated particle susceptible to be produced by the method of claim 13.

18. A cosmetic composition comprising, in a cosmetically acceptable medium, a synthetic polymer gel in accordance with claim 14, a dispersion of synthetic polymer gel particles in accordance with claim 15, a synthetic polymer gel particle in accordance with claim 16, and/or a coated particle in accordance with claim 17.

19. A cosmetic composition in accordance with claim 18, to be used as a body hygiene composition, for example in the form of deodorant stick; as a hair composition, for example as a styling stick or a make-up stick for the hair; as a make-up composition for the skin of the face or the body or for mucous membranes, for example as a lipstick, a foundation cast as a stick or a dish, a face powder, an eyeshadow, a fixing base to be applied over a conventional lipstick, a concealer stick, a lip gloss, an eyeliner, a mascara or temporary tattoo products; as a care composition for the skin or mucous membranes, for example as a lipcare balm or base, an ointment for the body or a dally care cream; as an antisun composition or a self-tanning composition; as skin-care compositions such as creams or facial washing gel.
